(19) Europäisches Patentamt
European Patent Office
Office européen des brevets

(11) Veröffentlichungsnummer: **0 418 362 B1**

(12) **EUROPÄISCHE PATENTSCHRIFT**

(45) Veröffentlichungstag der Patentschrift: **04.01.95**

(51) Int. Cl.[6]: **C09K 19/34**, C09K 19/30

(21) Anmeldenummer: **90905451.2**

(22) Anmeldetag: **31.03.90**

(86) Internationale Anmeldenummer:
**PCT/EP90/00513**

(87) Internationale Veröffentlichungsnummer:
**WO 90/12073 (18.10.90 90/24)**

(54) **TRIFLUORMETHYLCYCLOHEXAN-DERIVATE.**

(30) Priorität: **08.04.89 DE 3911621**
**09.09.89 DE 3930119**

(43) Veröffentlichungstag der Anmeldung:
**27.03.91 Patentblatt 91/13**

(45) Bekanntmachung des Hinweises auf die Patenterteilung:
**04.01.95 Patentblatt 95/01**

(84) Benannte Vertragsstaaten:
**DE FR GB IT NL**

(56) Entgegenhaltungen:
**WO-A-87/06602**

**I.M. Zalesskaya et al., Zh. Org Khim 1980, 16(6), 1194-202 (CA Vol. 93, 1980: 167704q.**

**A.N. Blatkitnyi et al., Zh. Org. Khim 1970, 6(10), 2055-60 (CA Vol. 74, 1971: 13282w).**

(73) Patentinhaber: **MERCK PATENT GmbH**
**Postfach,**
**Frankfurter Strasse 250**
**D-64271 Darmstadt (DE)**

(72) Erfinder: **REIFFENRATH, Volker**
**Jahnstr. 18**
**D-6101 Rossdorf (DE)**
Erfinder: **POETSCH, Eike**
**Am Buchwald 4**
**D-6109 Mühltal 6 (DE)**
Erfinder: **KURMEIER, Hans-Adolf**
**Hinter der Schule 3 a**
**D-6104 Seeheim-Jugenheim (DE)**
Erfinder: **WEBER, Georg**
**Wilhelm-Leuschner-Str. 38**
**D-6106 Erzhausen (DE)**
Erfinder: **HITTICH, Reinhard**
**Am Kirchberg 11**
**D-6101 Modautal 1 (DE)**
Erfinder: **KOMPTER, Hans-Michael**
**Mainzer Str. 14**
**D-6108 Weiterstadt (DE)**
Erfinder: **PLACH, Herbert**
**Wingertsbergstr. 5**
**D-6100 Darmstadt (DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft Trifluormethylcyclohexan-Derivate der Formel I

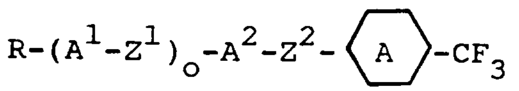

I

wobei

R ein unsubstituierter oder mit CN oder mit mindestens einem Halogen substituierter Alkyl- oder Alkenylrest mit bis zu 18 C-Atomen, worin eine oder mehrere nicht benachbarte $CH_2$-Gruppen durch einen Rest ausgewählt aus der Gruppe -O-, -S-, -CO-, -O-CO-, -CO-O- und -C≡C- ersetzt sein können,

$A^1$ und $A^2$ jeweils unabhängig voneinander

a) einen 1,4-Phenylenrest, worin eine oder zwei CH-Gruppen durch N ersetzt sein können,

b) einen 1,4-Cyclohexylenrest, worin eine oder zwei nicht benachbarte $CH_2$-Gruppen durch -O- oder -S- ersetzt sein können,

c) einen 1,4-Cyclohexenylen-, einen Piperidin-1,4-diyl-, einen 1,4-Bicyclo[2,2,2]-octylen- oder einen Naphthalin-2,6-diylrest

wobei die Reste a) und b) ein oder mehrfach durch Halogenatome, Cyano- und/oder Methylgruppen substituiert sein können,

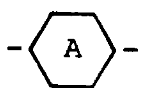 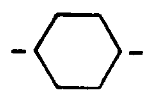 oder 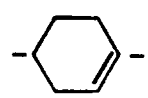

$Z^1$ und $Z^2$ jeweils unabhängig voneinander -CO-O-, -O-CO-, $-CH_2CH_2-$, $-CH(CN)-CH_2-$, $-CH_2-CH(CN)-$, -CH=CH-, $-OCH_2-$, $-CH_2O-$, -CH=N-, -N=CH-, -NO=N-, -N=NO-, -N=N- oder eine Einfachbindung, und

o 0, 1 oder 2 bedeuten.

Die Erfindung betrifft weiterhin die Verwendung dieser Verbindungen als Komponenten flüssigkristalliner Medien sowie Flüssigkristall- und elektrooptische Anzeigeelemente, die die erfindungsgemäßen flüssigkristallinen Medien enthalten.

Die Verbindungen der Formel I können als Komponenten flüssigkristalliner Medien verwendet werden, insbesondere für Displays, die auf dem Prinzip der verdrillten Zelle einschließlich deren hochverdrillten Varianten, wie z.B. STN oder SBE, dem Guest-Host-Effekt, dem Effekt der Deformation aufgerichteter Phasen oder dem Effekt der dynamischen Streuung beruhen, insbesondere für Matrix-Flüssigkristallanzeigen (MFK-Anzeigen).

Der Erfindung lag die Aufgabe zugrunde, neue stabile flüssigkristalline oder mesogene Verbindungen aufzufinden, die als Komponenten flüssigkristalliner Medien geeignet sind und insbesondere eine vergleichsweise geringe Viskosität besitzen sowie eine mittlere positive dielektrische Anisotropie.

Es wurde nun gefunden, daß Verbindungen der Formel I als Komponenten flüssigkristalliner Phasen vorzüglich geeignet sind. Insbesondere verfügen sie über vergleichsweise niedere Viskositäten. Mit ihrer Hilfe lassen sich stabile flüssigkristalline Phasen mit breitem Mesophasenbereich vorteilhaften Werten für die optische und dielektrische Anisotropie erhalten, welche sich gleichzeitig durch sehr günstige Werte für den spezifischen Widerstand auszeichnen. Hierdurch lassen sich insbesondere bei Medien für Matrix-Flüssigkristallanzeigen (MFK-Anzeigen) oder Supertwistdisplays deutliche Vorteile erzielen.

Ähnliche Verbindungen mit flüssigkristallinen Eigenschaften sind bereits bekannt, z.B. aus EP-A-0 0193 191, JP 59 078 129 oder JP 58 198 427. Bei diesen ist die Trifluormethylgruppe jedoch mit einem Phenylrest verknüpft daher besitzen sie im allgemeinen vergleichsweise unvorteilhafte Werte für die optische Anisotropie und sind für moderne Displayanwendungen den erfindungsgemäßen Verbindungen deutlich unterlegen.

Aus den JP 60-69 059 und JP 63 238 030 sind ähnliche Verbindungen bekannt, welche jedoch eine Gruppe

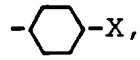

worin X CN, F oder Cl bedeutet, aufweisen.

Weiterhin sind Verbindungen bekannt, die eine Trifluormethylcyclohexylgruppe aufweisen, jedoch keinerlei mesogene Eigenschaften aufweisen. So werden z.B. in der JP 63 51 354 mit einer Trifluormethylgruppe substituierte Cyclohexancarbonsäuren sowie deren Darstellung beschrieben.

K.W. Baldrin, M.J.T. Robinson [Tetrahedron 33, 1663-1668 (1977)] führten Untersuchungen an verschiedenen 1-Trifluormethyl-3-substituierten Cyclohexanen durch.

Weiterhin werden in der DE-OS-30 22 818 in einer allgemeinen Formel unter anderem 4,4'-disubstituierte Bicyclohexylene als Mischungskomponenten von flüssigkristallinen Medien genannt, wobei einer der Substituenten auch eine Trifluormethylgruppe bedeuten kann.

Jedoch ist dort weder eine solche Verbindung noch deren Herstellung explizit beschrieben, noch kann der Fachmann die hervorragenden Eigenschaften dieser Verbindungen oder eine Herstellungsvorschrift dort entnehmen.

Mit der Bereitstellung von Verbindungen der Formel I wird außerdem ganz allgemein die Palette der flüssigkristallinen Substanzen, die sich unter verschiedenen anwendungstechnischen Gesichtspunkten zur Herstellung flüssigkristalliner Gemische eignen, erheblich verbreitert.

Die Verbindungen der Formel I besitzen einen breiten Anwendungsbereich. In Abhängigkeit von der Auswahl der Substituenten können diese Verbindungen als Basismaterialien dienen, aus denen flüssigkristalline Phasen zum überwiegenden Teil zusammengesetzt sind; es können aber auch Verbindungen der Formel I flüssigkristallinen Basismaterialien aus anderen Verbindungsklassen zugesetzt werden, um beispielsweise die dielektrische und/oder optische Anisotropie eines solchen Dielektrikums zu beeinflussen und/oder um dessen Schwellenspannung und/oder dessen Viskosität und/oder dessen spezifischen Widerstand zu optimieren.

Die Verbindungen der Formel I sind in reinem Zustand farblos und bilden flüssigkristalline Mesophasen in einem für die elektrooptische Verwendung günstig gelegenen Temperaturbereich. Chemisch, thermisch und gegen Licht sind sie stabil.

Gegenstand der Erfindung sind somit die Verbindungen der Formel I, insbesondere die Verbindungen der Formel I, worin die Reste $A^1$ und $A^2$ jeweils unabhängig voneinander einen Cyclohexylenrest, worin eine oder zwei nicht benachbarte $CH_2$-Gruppen durch -O- oder -S- ersetzt sein können bedeuten.

Gegenstand der Erfindung ist weiterhin die Verwendung dieser Verbindungen als Komponenten flüssigkristalliner Medien. Gegenstand der Erfindung sind ferner flüssigkristalline Medien mit einem Gehalt an mindestens einer Verbindung der Formel I sowie Flüssigkristallanzeigeelemente, insbesondere elektroopische Anzeigeelemente, insbesondere Matrix-Flüssigkristallanzeigen, die derartige Medien enthalten.

Der Einfachheit halber bedeuten im folgenden X $-CF_3$, Cyc einen 1,4-Cyclohexylen- oder einen 1,4-Cyclohexenylenrest, Dio einen 1,3-Dioxan-2,5-diylrest, Dit einen 1,3-Dithian-2,5-diylrest, Phe einen 1,4-Phenylenrest, Pyd einen Pyridin-2,5-diylrest, Pyr einen Pyrimidin-2,5-diylrest und Bi einen Bicyclo(2,2,2)-octylenrest, wobei Cyc und/oder Phe unsubstituiert oder ein- oder zweifach durch F oder CN substituiert sein können.

Die Verbindungen der Formel I umfassen dementsprechend Verbindungen mit zwei Ringen der Teilformeln Ia bis Ic:

$R-A^2-Cyc-X$ Ia

$A-A^2-Z^2-Cyc-X$ Ib

Verbindungen mit drei Ringen der Teilformeln Ic bis If:

$R-A^1-A^2-Cyc-X$ Ic

$R-A^1-Z^1-A^2-Cyc-X$ Id

R-$A^1$-$A^2$-$Z^2$-Cyc-X Ie

R-$A^1$-$Z^1$-$A^2$-$Z^2$-Cyc-X If

sowie Verbindungen mit vier Ringen der Teilformeln Ig bis Ih:

R-$A^1$-$A^1$-$A^2$-Cyc-X Ig

R-$A^1$-$Z^1$-$A^1$-$A^2$-Cyc-X Ih

R-$A^1$-$A^1$-$Z^1$-$A^2$-Cyc-X Ii

R-$A^1$-$A^1$-$A^2$-$Z^2$-Cyc-X Ij

R-$A^1$-$Z^1$-$A^1$-$Z^1$-$A^2$-Cyc-X Ik

R-$A^1$-$Z^1$-$A^1$-$A^2$-$Z^2$-Cyc-X Il

R-$A^1$-$A^1$-$Z^1$-$A^2$-$Z^2$-Cyc-X Im

R-$A^1$-$Z^1$-$A^1$-$Z^1$-$A^2$-$Z^2$-Cyc-X In

Darunter sind besonders diejenigen der Teilformeln Ia, Ib, Ic, Id, Ie, If, Ig, Ih, Ii und Il bevorzugt.

Bevorzugte Verbindungen der Formel I sind die 4-Trifluormethylbicyclohexyl-Derivate der Formel I1,

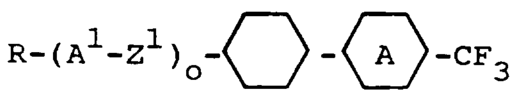

I1

worin R, $A^1$, $Z^1$, - A - und o die angegebene Bedeutung besitzen, insbesondere diejenigen worin o 0 oder 1 bedeutet.

Bevorzugte Verbindungen der Formel I sind weiterhin 2-(4-Trifluoromethylcyclohexyl)-dioxanderivate der Formel I2

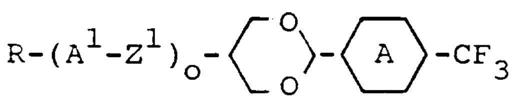

I2

worin R, $A^1$, $Z^1$, - A - und o die angegebene Bedeutung besitzen,
insbesondere diejenigen der Formel I2, worin o O ist und R eine Gruppe der Formel $(CH_2)_n$-X bedeutet, wobei

n 1 bis 10, und

X F, Cl, $CH=CH_2$, $CH=CH-C_mH_{2m+1}$, $O-C_mH_{2m+1}$, C≡CH oder H

bedeuten, worin

m 1 bis 10 ist.

Die bevorzugten Verbindungen der Teilformel Ia umfassen diejenigen der Teilformeln Iaa bis Iae:

R-Phe-Cyc-X Iaa

R-Cyc-Cyc-X Iab

R-Dio-Cyc-X Iac

R-Pyr-Cyc-X Iad

R-Pyd-Cyc-X Iae

Darunter sind diejenigen der Formeln Iaa und Iab besonders bevorzugt.

Die bevorzugten Verbindungen der Teilformel Ib umfassen diejenigen der Teilformeln Iba bis Ibl:

R-Phe-$CH_2CH_2$-Cyc-X Iba

R-Cyc-$CH_2CH_2$-Cyc-X Ibb

R-Cyc-$CH_2O$-Cyc-X Ibc

R-Phe-$CH_2O$-Cyc-X Ibd

R-Phe-CO-O-Cyc-X Ibe

R-Cyc-CO-O-Cyc-X Ibf

R-Cyc-C≡C-Cyc-X Ibg

R-Phe-C≡C-Cyc-X Ibh

R-Phe-$OCH_2$-Cyc-X Ibi

R-Cyc-$OCH_2$-Cyc-X Ibj

R-Cyc-O-CO-Cyc-X Ibk

R-Phe-O-CO-Cyc-X Ibl

Die bevorzugten Verbindungen der Teilformel Ic umfassen diejenigen der Teilformeln Ica bis Ich:

R-Cyc-Cyc-Cyc-X Ica

R-Phe-Cyc-Cyc-X Icb

R-Phe-Phe-Cyc-X Icc

R-Cyc-Phe-Cyc-X Icd

R-Pyd-Phe-Cyc-X Ice

R-Pyd-Cyc-Cyc-X Icf

R-Pyr-Cyc-Cyc-X Icg

R-Pyr-Phe-Cyc-X Ich

Darunter sind diejenigen der Formeln Ica, Icb und Icc besonders bevorzugt.

Die bevorzugten Verbindungen der Teilformel Id umfassen diejenigen der Teilformeln Ida bis Idi:

R-Phe-$Z^1$-Phe-Cyc-X Ida

R-Phe-$Z^1$-Cyc-Cyc-X Idb

R-Cyc-$Z^1$-Cyc-Cyc-X Idc

R-Pyr-$Z^1$-Cyc-Cyc-X Idd

R-Pyd-$Z^1$-Cyc-Cyc-X Ide

R-Pyd-$Z^1$-Phe-Cyc-X Idf

R-Pyr-$Z^1$-Phe-Cyc-X Idg

R-Cyc-$Z^1$-Dio-Cyc-X Idh

R-Phe-$Z^1$-Dio-Cyc-X Idi

Die bevorzugten Verbindungen der Teilformel Ie umfassen diejenigen der Teilformeln Iea bis Iei:

R-Phe-Phe-$Z^2$-Cyc-X Iea

R-Phe-Cyc-$Z^2$-Cyc-X Ieb

R-Cyc-Cyc-$Z^2$-Cyc-X Iec

R-Cyc-Phe-$Z^2$-Cyc-X Ied

R-Pyr-Phe-$Z^2$-Cyc-X Iee

R-Pyd-Phe-$Z^2$-Cyc-X Ief

R-Phe-Pyr-$Z^2$-Cyc-X Ieg

R-Phe-Pyd-$Z^2$-Cyc-X Ieh

R-Phe-Dio-$Z^2$-Cyc-X Iei

Die bevorzugten Verbindungen der Teilformel If umfassen diejenigen der Teilformeln Ifa bis Ifg

R-Phe-$Z^1$-Phe-$Z^2$-Cyc-X Ifa

R-Phe-$Z^1$-Cyc-$Z^2$-Cyc-X Ifb

R-Cyc-$Z^1$-Cyc-$Z^2$-Cyc-X Ifc

R-Cyc-$Z^1$-Phe-$Z^2$-Cyc-X Ifd

R-Cyc-$Z^1$-Bi-$Z^2$-Cyc-X Ife

R-Cyc-$Z^1$-Dio-$Z^2$-Cyc-X Iff

R-Dio-$Z^1$-Cyc-$Z^2$-Cyc-X Ifg

In den Verbindungen der vor- und nachstehenden Formeln bedeutet Cyc-X bevorzugt eine 4-Trifluormethylcyclohexylgruppe, insbesondere eine trans-4-Trifluormethylcyclohexylgruppe.

In den Verbindungen der Teilformeln Ib, Id, Ie, If sowie Ih bis In, die eine oder mehrere Brücken $Z^1$ und/oder $Z^2$ aufweisen, bedeuten diese Brückenglieder vorzugsweise -CO-O-, -O-CO-, $-CH_2CH_2-$, $CH_2O$-, $-OCH_2-$ oder -C≡C-, insbesondere $-CH_2CH_2-$, -CO-O- oder -O-CO-.

R bedeutet vorzugsweise Alkyl, ferner Alkoxy, F, Cl oder CN. $A^1$ und/oder $A^2$ bedeuten bevorzugt Phe, Cyc, Che, Pyr oder Dio. Bevorzugt enthalten die Verbindungen der Formel I nicht mehr als einen der Reste Bi, Pyd, Pyr, Dio oder Dit.

Bevorzugt sind auch Verbindungen der Formel I sowie aller Teilformeln, in denen $A^1$, $A^2$, $A^3$ und/oder $A^4$ ein- oder zweifach durch F oder einfach durch CN substituiertes 1,4-Phenylen bedeutet. Insbesondere sind dies 2-Fluor-1,4-phenylen, 3-Fluor-1,4-phenylen und 2,3-Difluor-1,4-phenylen sowie 2-Cyan-1,4-phenylen und 3-Cyan-1,4-phenylen.

$Z^1$ und $Z^2$ bedeuten bevorzugt eine Einfachbindung, -CO-O-, -O-CO- und -$CH_2CH_2$-, in zweiter Linie bevorzugt -$CH_2O$- und -$OCH_2$-.

Falls R Halogen bedeutet, so bedeutet er vorzugsweise F, Cl, Br, ferner auch I.

Falls R einen Alkylrest oder einen Alkoxyrest bedeutet, so kann dieser geradkettig oder verzweigt sein. Vorzugsweise ist er geradkettig, hat 2, 3, 4, 5, 6 oder 7 C-Atome und bedeutet demnach bevorzugt Ethyl, Propyl, Butyl, Pentyl, Hexyl, Heptyl, Ethoxy, Propoxy, Butoxy, Pentoxy, Hexoxy oder Heptoxy, ferner Methyl, Octyl, Nonyl, Decyl, Undecyl, Dodecyl, Tridecyl, Tetradecyl, Pentadecyl, Methoxy, Octoxy, Nonoxy, Decoxy, Undecoxy, Dodecoxy, Tridecoxy oder Tetradecoxy.

Oxaalkyl bedeutet vorzugsweise geradkettiges 2-Oxapropyl (= Methoxymethyl), 2- (= Ethoxymethyl) oder 3-Oxabutyl (= 2-Methoxyethyl), 2-, 3- oder 4-Oxapentyl, 2-, 3-, 4- oder 5-Oxahexyl, 2-, 3-, 4-, 5- oder 6-Oxaheptyl, 2-, 3-, 4-, 5-, 6- oder 7-Oxaoctyl, 2-, 3-, 4-, 5-, 6-, 7- oder 8-Oxanonyl, 2-, 3-, 4-, 5-, 6-, 7-, 8- oder 9-Oxadecyl.

Falls R einen Alkenylrest bedeutet, so kann dieser geradkettig oder verzweigt sein. Vorzugsweise ist er geradkettig und hat 2 bis 10 C-Atome. Er bedeutet demnach besonders Vinyl, Prop-1-, oder Prop-2-enyl, But-1-, 2- oder But-3-enyl, Pent-1-, 2-, 3- oder Pent-4-enyl, Hex-1-, 2-, 3-, 4- oder Hex-5-enyl, Hept-1-, 2-, 3-, 4-, 5- oder Hept-6-enyl, Oct-1-, 2-, 3-, 4-, 5-, 6- oder Oct-7-enyl, Non-1-, 2-, 3-, 4-, 5-, 6-, 7- oder Non-8-enyl, Dec-1-, 2-, 3-, 4-, 5-, 6-, 7-, 8- oder Dec-9-enyl.

Falls R einen Alkylrest bedeutet, in dem eine $CH_2$-Gruppe durch -O- und eine durch -CO- ersetzt ist, so sind diese bevorzugt benachbart. Somit beeinhalten diese eine Acyloxygruppe -CO-O- oder eine Oxycarbonylgruppe -O-CO-. Vorzugsweise sind diese geradkettig und haben 2 bis 6 C-Atome. Sie bedeuten demnach besonders Acetyloxy, Propionyloxy, Butyryloxy, Pentanoyloxy, Hexanoyloxy, Acetyloxymethyl, Propionyloxymethyl, Butyryloxymethyl, Pentanoyloxymethyl, 2-Acetyloxyethyl, 2-Propionyloxyethyl, 2-Butyryloxyethyl, 3-Acetyloxypropyl, 3-Propionyloxypropyl, 4-Acetyloxybutyl, Methoxycarbonyl, Ethoxycarbonyl, Propoxycarbonyl, Butoxycarbonyl, Pentoxycarbonyl, Methoxycarbonylmethyl, Ethoxycarbonylmethyl, Propoxycarbonylmethyl, Butoxycarbonylmethyl, 2-(Methoxycarbonyl)ethyl, 2-(Ethoxacarbonyl)ethyl, 2-(Propoxycarbonyl)ethyl, 3-(Methoxycarbonyl)propyl, 3-(Ethoxycarbonyl)propyl, 4-(Methoxycarbonyl)-butyl.

Falls R einen Alkenylrest bedeutet, in dem eine $CH_2$-Gruppe durch CO oder CO-O oder O-CO-ersetzt ist, so kann dieser geradkettig oder verzweigt sein. Vorzugsweise ist er geradkettig und hat 4 bis 13 C-Atome. Er bedeutet demnach besonders Acryloyloxymethyl, 2-Acryloyloxyethyl, 3-Acryloyloxypropyl, 4-Acryloyloxybutyl, 5-Acryloyloxypentyl, 6-Acryloyloxyhexyl, 7-Acryloyloxyheptyl, 8-Acryloyloxyoctyl, 9-Acryloyloxynonyl, 10-Acryloyloxydecyl, Methacryloyloxymethyl, 2-Methacryloyloxyethyl, 3-Methacryloyloxypropyl, 4-Methacryloyloxybutyl, 5-Methacryloyloxypentyl, 6-Methacryloyloxyhexyl, 7-Methacryloyloxyheptyl, 8-Methacryloyloxyoctyl, 9-Methacryloyloxynonyl.

Verbindungen der Formel I, die über für Polymerisationsreaktionen geeignete Flügelgruppen R verfügen, eignen sich zur Darstellung flüssigkristalliner Polymerer.

Verbindungen der Formeln I mit verzweigten Flügelgruppen R können gelegentlich wegen einer besseren Löslichkeit in den üblichen flüssigkristallinen Basismaterialien von Bedeutung sein, insbesondere aber als chirale Dotierstoffe, wenn sie optisch aktiv sind. Smektische Verbindungen dieser Art eignen sich als Komponenten für ferroelektrische Materialien.

Verbindungen der Formel I mit $S_A$-Phasen eignen sich beispielsweise für thermisch adressierte Displays.

Verzweigte Gruppen dieser Art enthalten in der Regel nicht mehr als eine Kettenverzweigung. Bevorzugte verzweigte Reste R sind Isopropyl, 2-Butyl (= 1-Methylpropyl), Isobutyl (= 2-Methylpropyl), 2-Methylbutyl, Isopentyl (= 3-Methylbutyl), 2-Methylpentyl, 3-Methylpentyl, 2-Ethylhexyl, 2-Propylpentyl, Isopropoxy, 2-Methylpropoxy, 2-Methylbutoxy, 3-Methylbutoxy, 2-Methylpentoxy, 3-Methylpentoxy, 2-Ethylhexoxy, 1-Methylhexoxy, 1-Methylheptoxy, 2-Oxa-3-methylbutyl, 3-Oxa-4-methylpentyl, 4-Methylhexyl, 2-Nonyl, 2-Decyl, 2-Dodecyl, 6-Methyloctoxy, 6-Methyloctaroyloxy, 5-Methylheptyloxycarbonyl, 2-Methylbutyryloxy, 3-Methylvaleryloxy, 4-Methylhexanoyloxy, 2-Chlorpropionyloxy, 2-Chlor-3-methylbutyryloxy, 2-Chlor-4-methylvaleryloxy, 2-Chlor-3-methylvaleryloxy, 2-Methyl-3-oxapentyl, 2-Methyl-3-oxahexyl.

Falls R einen Alkylrest darstellt, in dem zwei oder mehr $CH_2$-Gruppen durch -O- und/oder -CO-O-ersetzt sind, so kann dieser geradkettig oder verzweigt sein. Vorzugsweise ist er verzweigt und hat 3 bis 12 C-Atome. Er bedeutet demnach besonders Bis-carboxy-methyl, 2,2-Bis-carboxy-ethyl, 3,3-Bis-carboxy-propyl, 4,4-Bis-carboxy-butyl, 5,5-Bis-carboxy-pentyl, 6,6-Bis-carboxy-hexyl, 7,7-Bis-carboxy-heptyl, 8,8-Bis-carboxy-octyl, 9,9-Bis-carboxy-nonyl, 10,10-Bis-carboxy-decyl, Bis-(methoxycarbonyl)-methyl, 2,2-Bis-(methoxycarbonyl)-ethyl, 3,3-Bis-(methoxycarbonyl)-propyl, 4,4-Bis-(methoxycarbonyl)-butyl, 5,5-Bis-(methoxycarbonyl)-pentyl, 6,6-Bis-(methoxycarbonyl)-hexyl, 7,7-Bis-(methoxycarbonyl)-heptyl, 8,8-Bis-(methoxycarbonyl)-octyl, Bis-(ethoxycarbonyl)-methyl, 2,2-Bis-(ethoxycarbonyl)-ethyl, 3,3-Bis-(ethoxycarbonyl)-propyl, 4,4-Bis-(ethoxycarbonyl)-butyl, 5,5-Bis-(ethoxycarbonyl)-hexyl.

Verbindungen der Formel I, die über für Polykondensationen geeignete Flügelgruppen R verfügen, eignen sich zur Darstellung flüssigkristalliner Polykondensate.

Formel I umfaßt sowohl die Racemate dieser Verbindungen als auch die optischen Antipoden sowie deren Gemische.

Unter diesen Verbindungen der Formel I sowie den Unterformeln sind diejenigen bevorzugt, in denen mindestens einer der darin enthaltenden Reste eine der angegebenen bevorzugten Bedeutungen hat.

In den Verbindungen der Formel I sind diejenigen Stereoisomeren bevorzugt, in denen die Ringe Cyc und Piperidin trans-1,4-disubstituiert sind. Diejenigen der vorstehend genannten Formeln, die eine oder mehrere Gruppen Pyd, Pyr und/oder Dio enthalten, umschließen jeweils die beiden 2,5-Stellungsisomeren.

Die 1,4-Cyclohexenylen-Gruppe hat vorzugsweise folgende Strukturen:

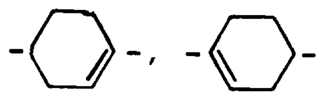

Die Verbindungen der Formel I werden nach an sich bekannten Methoden dargestellt, wie sie in der Literatur (z.B. in den Standardwerken wie Houben-Weyl, Methoden der Organischen Chemie, Georg-Thieme-Verlag, Stuttgart Bd IX, S. 867 ff.) beschrieben sind, und zwar unter Reaktionsbedingungen, die für die genannten Umsetzungen bekannt und geeignet sind. Dabei kann man auch von an sich bekannten, hier nicht näher erwähnten Varianten Gebrauch machen.

Die erfindungsgemäßen Trifluormethylcyclohexylverbindungen der Formel I können z.B. hergestellt werden, indem man die der Formel I entsprechenden Cyclohexancarbonsäuren mit Dialkylaminoschwefeltrifluoriden, beispielsweise DAST (Diethylaminoschwefeltrifluorid) [W.J. Middleton J.Org. Chem. 40, 574, (1975)] oder Schwefeltetrafluorid [A. Haus, M. Spitzer, M. Lieb, Chem. Ber. 121 (1988), 1329] umsetzt, gemäß dem Schema

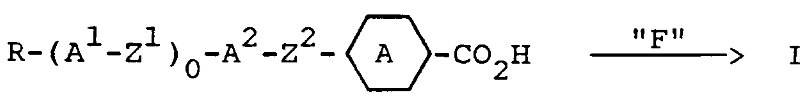


Weiterhin ist es möglich, die Verbindungen der Formel I aus den entsprechenden Trifluorphenylverbindungen durch katalytische Hydrierung herzustellen [z.B. analog K.W. Baldrin, M.J.T. Robinson, Tetrahedron 33, 1663-1668 (1977)]

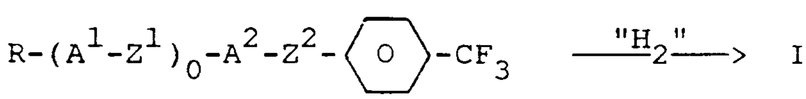


Darüberhinaus können die Verbindungen der Formel I durch Umsetzung der entsprechenden 4-substituierten -1-Trifluormethylcyclohexane der Formel II

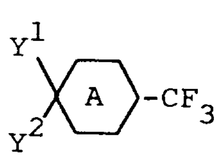

II

worin

$Y^1$ $-(CH_2)_n-Y^3$, -CHO oder CN,

$Y^2$ H,

oder

$Y^1$ und $Y^2$ zusammengenommen = O

$Y^3$ OH, Halogen, und

n 0, 1 oder 2 bedeutet,

mit dem entsprechend substituierten Verbindungen der Formel III $R-(A^1-Z^1)_o-A^2-Z^3$ III, hergestellt werden.

Die Verbindungen der Formel II sind teilweise bekannt: I. M Zalesskaya et al., Zh. Org. Khim 1980, 16-(6), 1194-202 (CA Vol. 93, 1980: 167704q) offenbaren 4-Trifluorcyclohexanol und 4-Trifluorcyclohexanon; A.N. Blatkitnyi et al., Zh. Org. Khim 1970, 6(10), 2055-60 (CA Vol. 74, 1971: 13282w) offenbaren 4-Trifluorbromcyclohexan.

Es gibt dort keinerlei Hinweis, daß solche Verbindungen zur Herstellung mesogener Verbindungen eingesetzt werden können.

Die neuen Verbindungen der Formel II sind ebenfalls Gegenstand der Erfindung.

Die Verbindungen der Formel I, worin $Z^2$ $CH_2O$ oder $OCH_2$ bedeutet, erhält man z.B. durch Veretherung der Verbindungen der Formel II, worin $Y^1$ $(CH_2)_n-Y^3$ bedeutet, mit den Verbindungen der Formel III, worin $Z^3$ $CH_2OH$ bzw. OH bedeutet.

Die Verbindungen der Formel I, worin $Z^2$ $CH_2CH_2$ bedeutet, können z.B. durch Umsetzung der Verbindungen der Formel III, worin $Z^3$ $CH_2$ Met und Met Li, Na, K, $P^{\oplus}$ $(C_6H_5)_3$ oder P (O) $(OC_2H_5)_2$ bedeutet mit den Verbindungen der Formel II, worin $Y^1$ CN bzw. CHO bedeutet, und durch anschließende Reduktion des so erhaltenen Ketons bzw. Ethylenderivats, hergestellt werden.

Die Verbindungen der Formel I, worin $Z^2$ eine Einfachbindung bedeutet, können z.B. durch Umsetzung der Verbindungen der Formel III, worin $Z^3$ Met bedeutet, mit dem Verbindungen der Formel II, worin $Y^1$ und $Y^2$ zusammengenommen = O bedeuten, anschließende Dehydratisierung und gegebenenfalls katalytische Hydrierung des so erhaltenen Cyclohexen-Derivats erhalten werden.

Weiterhin können die Verbindungen der Formel I, in dem man die der Formel I entsprechenden Cyclohexanone mit Trifluormethyl-Met, worin Met Li, Na, K, MgBr oder MgCl bedeutet bzw. mit Trimethyl-(trifluormethyl)-silan in Gegenwart einer Lewis-Säure, wie z.B. Titantetrachlorid oder Zinkbromid, oder eines Fluorids, wie z.B. Tetrabutylammoniumfluorid oder Cäsiumfluorid, z.B. nach C.F. Olah et al. J.Am.Chem.Soc. 111, 393-395 (1989) umsetzt und das entsprechende Trifluormethylhydrin in Gegenwart eines Dehydratisierungsreagenzes dehydratisiert.

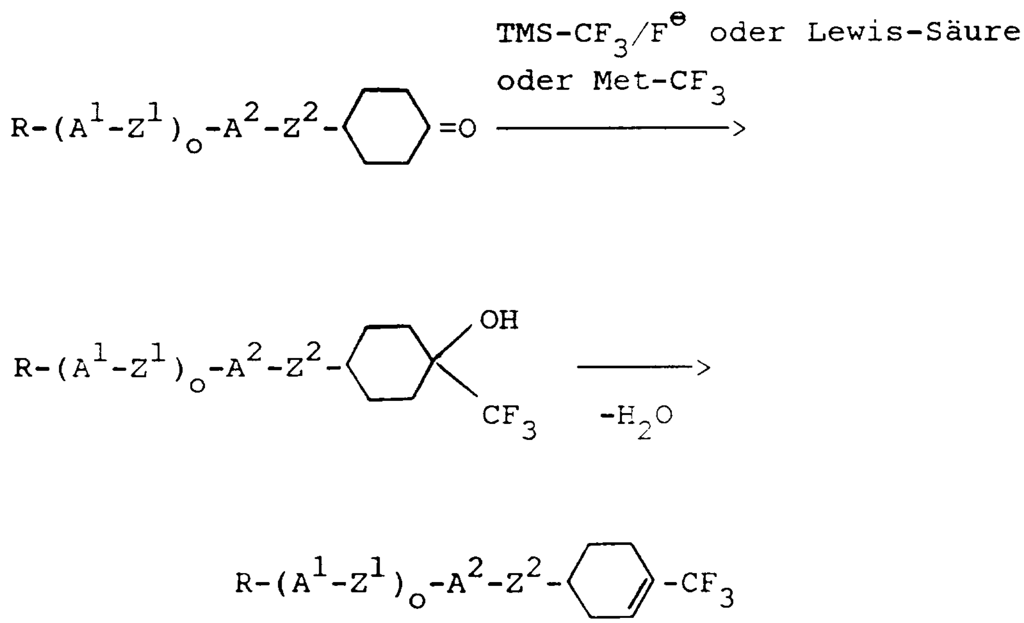


Geeignete Dehydratisierungsmittel sind z.B. $P_2O_5$, $POCl_3$, $PCl_3$, $PCl_5$, $COCl_2$ und DAST.

Darüber hinaus lassen sich die Verbindungen der Formel I, worin

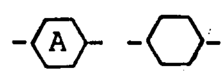

bedeutet, eine durch Lewis-Säure katalysierte Diels-Alder Kondensation von 2-Trifluormethylbutadien an die entsprechenden Vinyl-Verbindungen (z.B. nach T. Kojuma, T. Inuhai, J.Org.Chem. 35 (5), 1342-1348 (1970))

gemäß Schema II herstellen.

Schema II

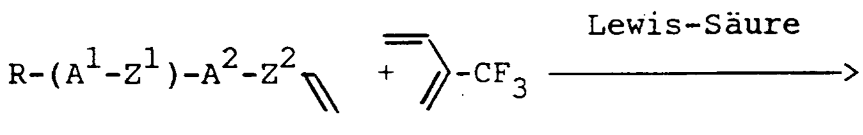


I

Vorzugsweise werden nach diesem Verfahren die Verbindungen der Formel I, worin $Z^2$-O-CO bedeutet, insbesondere auch die Zwischenprodukte der Formel IIa,

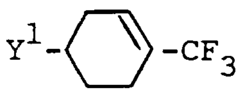

IIa

worin
- $Y^1$ CHO, CN oder COOR°, und
- R° H oder Alkyl mit 1 bis 6 C-Atomen

bedeuten,
hergestellt.

2-Trifluorbutadien ist erhältlich aus 1,1,1-Trifluoraceton nach T. Inuhai, M. Kasai, J.Org.Chem. 30 3567 (1965).

Die 2-(4-Trifluormethylcyclohexyl)dioxane der Formel I2 werden gemäß Schema III durch Kondensation von entsprechend substituierten Propandiolen mit 4-Formyl-trifluormethylcyclohexan hergestellt.

Schema III

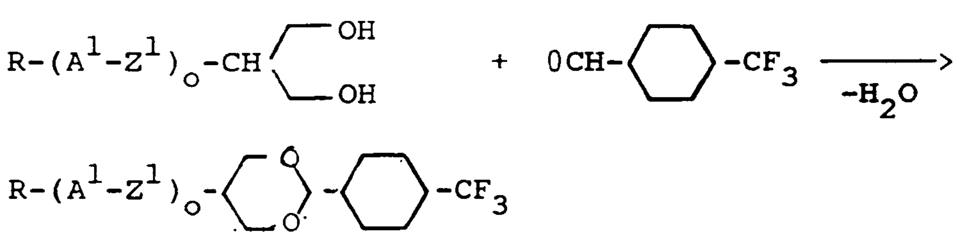


Die entsprechend substituierten Propandiole werden gemäß Schema IV durch Alkylierung von Malonestern bzw. in Analogie zu dem in DE-32 27 916 beschriebenen Verfahren hergestellt:

Schema IV:

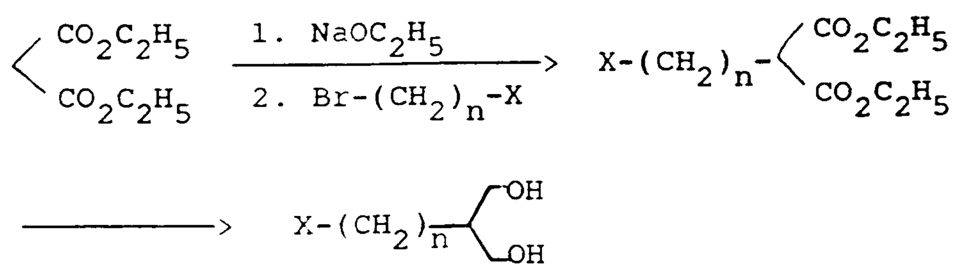


Ausgangstoffe können gewünschtenfalls auch in situ gebildet werden, derart, daß man sie aus dem Reaktionsgemisch nicht isoliert, sondern sofort weiter zu den Verbindungen der Formel I umsetzt.

So können die Verbindungen der Formel I hergestellt werden, indem man eine Verbindung, die sonst der Formel I entspricht, aber an Stelle von H-Atomen eine oder mehrere reduzierbare Gruppen und/oder C-C-Bindungen enthält, reduziert.

Als reduzierbare Gruppen kommen vorzugsweise Carbonylgruppen in Betracht, insbesondere Ketogruppen, ferner z.B. freie oder veresterte Hydroxygruppen oder aromatisch gebundene Halogenatome. Bevorzugte Ausgangsstoffe für die Reduktion entsprechen der Formel I, können aber an Stelle eines Cyclohexanringes einen Cyclohexenring, Cyclohexanonring oder einen Phenylring und/oder an Stelle einer $-CH_2CH_2-$Gruppe eine -CH=CH-Gruppe und/oder an Stelle einer $-CH_2$-Gruppe eine -CO-Gruppe und/oder an Stelle eines H-Atoms eine freie oder eine funktionell (z.B. in Form ihres p-Toluolsulfonats) abgewandelte OH-Gruppe enthalten.

Die Reduktion kann z.B. erfolgen durch katalytische Hydrierung bei Temperaturen zwischen etwa 0° und etwa 200° sowie Drucken zwischen etwa 1 und 200 bar in einem inerten Lösungsmittel, z.B. einem Alkohol wie Methanol, Ethanol oder Isopropanol, einem Ether wie Tetrahydrofuran (THF) oder Dioxan, einem Ester wie Ethylacetat, einer Carbonsäure wie Essigsäure oder einem Kohlenwasserstoff wie Cyclohexan. Als Katalysatoren eignen sich zweckmäßig Edelmetalle wie Pt oder Pd, die in Form von Oxiden (z.B. $PtO_2$, PdO), auf einem Träger (z.B. Pd auf Kohle, Calciumcarbonat oder Strontiumcarbonat) oder in feinverteilter Form eingesetzt werden können.

Ketone können auch nach den Methoden von Clemmensen (mit Zink, amalgamiertem Zink oder Zinn und Salzsäure, zweckmäßig in wäßrig-alkoholischer Lösung oder in heterogener Phase mit Wasser/Toluol bei Temperaturen zwischen etwa 80 und 120°) oder Wolff-Kishner (mit Hydrazin, Zweckmäßig in Gegenwart von Alkali wie KOH oder NaOH in einem hochsiedenden Lösungsmittel wie Diethylenglykol oder Triethylenglykol bei Temperaturen zwischen etwa 100 und 200°) zu den entsprechenden Verbindungen der Formel I, die Alkylgruppen und/oder $-CH_2CH_2$-Brücken enthalten, reduziert werden.

Weiterhin sind Reduktionen mit komplexen Hydriden möglich. Beispielsweise können Arylsulfonyloxygruppen mit $LiAlH_4$ reduktiv entfernt werden, insbesondere p-Toluolsulfonyloxymethylgruppen zu Methylgruppen reduziert werden, zweckmäßig in einem inerten Lösungsmittel wie Diethylether oder THF bei Temperaturen zwischen etwa 0 und 100°. Doppelbindungen können mit $NaBH_4$ oder Tributylzinnhydrid in Methanol hydriert werden.

Verbindungen der Formel I, die ansonsten der Formel I entsprechen, aber an Stelle von 1,4-Phenylenresten 1,4-Cyclohexenylenreste besitzen, können zum Beispiel mit DDQ (Dichlordicyanobenzochinon) in einem geeigneten Lösungsmittel oxidiert werden.

Ester der Formel I können auch durch Veresterung entsprechender Carbonsäuren (oder ihrer reaktionsfähigen Derivate) mit Alkoholen bzw. Phenolen (oder ihren reaktionsfähigen Derivaten) oder nach der DCC-Methode (DCC = Dicyclohexylcarbodiimid) erhalten werden.

Die entsprechenden Carbonsäuren und Alkohole bzw. Phenole sind bekannt oder können in Analogie zu bekannten Verfahren hergestellt werden.

Als reaktionsfähige Derivate der genannten Carbonsäuren eignen sich insbesondere die Säurehalogenide, vor allem die Chloride und Bromide, ferner die Anhydride, z.B. auch gemischte Anhydride, Azide oder Ester, insbesondere Alkylester mit 1 - 4 C-Atomen in der Alkylgruppe.

Als reaktionsfähige Derivate der genannten Alkohole bzw. Phenole kommen insbesondere die entsprechenden Metallalkoholate bzw. Phenolate, vorzugsweise eines Alkalimetalls wie Natrium oder Kalium, in Betracht.

Die Veresterung wird vorteilhaft in Gegenwart eines inerten Lösungsmittels durchgeführt. Gut geeignet sind insbesondere Ether wie z.B. Diethylether, Di-n-butylether, THF, Dioxan oder Anisol, Ketone wie z. B. Aceton, Butanon oder Cyclohexanon, Amide wie z. B. DMF oder Phosphorsäurehexamethyltriamid, Kohlenwasserstoffe wie z.B. Benzol, Toluol oder Xylol, Halogenkohlenwasserstoffe wie z. B. Tetrachlorkohlenstoff, Dichlormethan oder Tetrachlorethylen und Sulfoxide wie z. B. Dimethylsulfoxid oder Sulfolan.

Zur Herstellung von Nitrilen der Formel I können entsprechende Säureamide, z.B. solche, in denen an Stelle des Restes CN eine $CONH_2$-Gruppe steht, dehydratisiert werden. Die Amide sind z.B. aus entsprechenden Estern oder Säurehalogeniden durch Umsetzung mit Ammoniak erhältlich. Als wasserabspaltende Mittel eignen sich beispielsweise anorganische Säurechloride wie $SOCl_2$, $PCl_3$, $PCl_5$, $POCl_3$, $SO_2Cl_2$, $COCl_2$, ferner $P_2O_5$, $P_2S_5$, $AlCl_3$ (z.B. als Doppelverbindung mit NaCl), aromatische Sulfonsäuren und Sulfonsäurehalogenide. Man kann dabei in Gegenwart oder Abwesenheit eines inerten Lösungsmittels bei Temperaturen zwischen etwa 0° und 150° arbeiten; als Lösungsmittel kommen z.B. Basen wie Pyridin oder Triethylamin, aromatische Kohlenwasserstoffe wie Benzol, Toluol oder Xylol oder Amide wie DMF in Betracht.

Zur Herstellung der vorstehend genannten Nitrile der Formel I kann man auch entsprechende Säurehalogenide, vorzugsweise die Chloride, mit Sulfamid umsetzen, zweckmäßig in einem inerten Lösungsmittel wie z. B. Tetramethylensulfon bei Temperaturen zwischen etwa 80° und 150°, vorzugsweise bei 120°. Nach üblicher Aufarbeitung kann man direkt die Nitrile isolieren.

Ether der Formel I sind durch Veretherung entsprechender Hydroxyverbindungen, vorzugsweise entsprechender Phenole, erhältlich, wobei die Hydroxyverbindung zweckmäßig zunächst in ein entsprechendes Metallderivat, z.B. durch Behandeln mit NaH, $NaNH_2$, NaOH, KOH, $Na_2CO_3$ oder $K_2CO_3$ in das entsprechende Alkalimetallalkoholat oder Alkalimetallphenolat übergeführt wird. Dieses kann dann mit dem entsprechenden Alkylhalogenid, -sulfonat oder Dialkylsulfat umgesetzt werden, zweckmäßig in einem inerten Lösungsmittel wie z. B. Aceton, 1,2-Dimethoxyethan, DMF oder Dimethylsulfoxid oder auch mit einem Überschuß an wäßriger oder wäßrig-alkoholischer NaOH oder KOH bei Temperaturen zwischen etwa 20° und 100°.

Zur Herstellung von Nitrilen der Formel I können auch entsprechende Chlor-, Brom- oder Jodverbindungen der Formel I mit einem Cyanid umgesetzt werden, vorzugsweise mit einem Metallcyanid wie z. B. NaCN, KCN oder $Cu_2(CN)_2$,z. B. in Gegenwart von Pyridin in einem inerten Lösungsmittel wie z. B. DMF oder N-Methylpyrrolidon bei Temperaturen zwischen 20° und 200°.

Verbindungen der Formel I, worin $A^1$ durch mindestens ein F-Atom und/oder eine CN-Gruppe substituiert ist, können auch aus den entsprechenden Diazoniumsalzen durch Austausch der Diazoniumgruppe gegen ein Fluoratom oder gegen eine CN-Gruppe, z.B. nach den Methoden von Schiemann oder Sandmeyer, erhalten werden.

Dioxanderivate bzw. Dithianderivate der Formel I werden zwecksmäßig durch Reaktion eines entsprechenden Aldehyds (oder eines seiner reaktionsfähigen Derivate) mit einem entsprechenden 1,3-Diol (oder einem seiner reaktionsfähigen Derivate) bzw. einem entsprechenden 1,3-Dithiol hergestellt, vorzugsweise in Gegenwart eines inerten Lösungsmittels wie z.B. Benzol oder Toluol und/oder eines Katalysators, z.B. einer starken Säure wie Schwefelsäure, Benzol- oder p-Toluolsulfonsäure, bei Temperaturen zwischen etwa 20° und etwa 150°, vorzugsweise zwischen 80° und 120°. Als reaktionsfähige Derivate der Ausgangsstoffe eignen sich in erster Linie Acetale.

Die genannten Aldehyde und 1,3-Diole bzw. 1,3-Dithiole sowie ihre reaktionsfähigen Derivate sind zum Teil bekannt, zum Teil können sie ohne Schwierigkeiten nach Standardverfahren der Organischen Chemie aus literaturbekannten Verbindungen hergestellt werden. Beispielsweise sind die Aldehyde durch Oxydation entsprechender Alkohole oder durch Reduktion von Nitrilen oder entsprechenden Carbonsäuren oder ihrer Derivate, die Diole durch Reduktion entsprechender Diester und die Dithiole durch Umsetzung entsprechender Dihalogenide mit NaSH erhältlich.

Die erfindungsgemäßen flüssigkristallinen Medien enthalten vorzugsweise neben einer oder mehreren erfindungsgemäßen Verbindungen als weitere Bestandteile 2 bis 40, insbesondere 4 bis 30 Komponenten. Ganz besonders bevorzugt enthalten diese Medien neben einer oder mehreren erfindungsgemäßen Verbindungen 7 bis 25 Komponenten. Diese weiteren Bestandteile werden vorzugsweise ausgewählt aus nematischen oder nematogenen (monotropen oder isotropen) Substanzen, insbesondere Substanzen aus den Klassen der Azoxybenzole, Benzylidenaniline, Biphenyle, Terphenyle, Phenyl- oder Cyclohexylbenzoate, Cyclohexan-carbonsäurephenyl- oder cyclohexyl-ester, Phenyl- oder Cyclohexylester der Cyclohexylbenzoesäure, Phenyl- oder Cyclohexylester der Cyclohexylcyclohexancarbonsäure, Cyclohexylphenylester der Benzoesäure, der Cyclohexancarbonsäure, bzw. der Cyclohexylcyclohexancarbonsäure, Phenylcyclohexane, Cyclohexylbiphenyle, Phenylcyclohexylcyclohexane, Cyclohexylcyclohexane, Cyclohexylcyclohexene, Cyclohexylcyclohexylcyclohexene, 1,4-Bis-cyclohexylbenzole, 4,4'-Bis-cyclohexylbiphenyle, Phenyl- oder

Cyclohexylpyrimidine, Phenyl- oder Cyclohexylpyridine, Phenyl- oder Cyclohexyldioxane, Phenyl- oder Cyclohexyl-1,3-dithiane, 1,2-Diphenylethane, 1,2-Dicyclohexylethane, 1-Phenyl-2-cyclohexylethane, 1-Cyclohexyl-2-(4-phenyl-cyclohexyl)-ethane, 1-Cyclohexyl-2-biphenylylethane, 1-Phenyl-2-cyclohexylphenylethane, gegebenenfalls halogenierten Stilbene, Benzylphenylether, Tolane und substituierten Zimtsäuren. Die 1,4-Phenylengruppen in diesen Verbindungen können auch fluoriert sein.

Die wichtigsten als weitere Bestandteile erfindungsgemäßer Medien in Frage kommenden Verbindungen lassen sich durch die Formeln 1, 2, 3, 4 und 5 charakterisieren:

R'-L-E-R'' 1

R'-L-COO-E-R'' 2

R'-L-OOC-E-R'' 3

R'-L-$CH_2CH_2$-E-R'' 4

R'-L-C≡C-E-R'' 5

In den Formeln 1, 2, 3, 4 und 5 bedeuten L und E, die gleich oder verschieden sein können, jeweils unabhängig voneinander einen bivalenten Rest aus der aus -Phe-, -Cyc-, -Phe-Phe-, -Phe-Cyc-, -Cyc-Cyc-, -Pyr-, -Dio-, -G-Phe- und -G-Cyc- sowie deren Spiegelbilder gebildeten Gruppe, wobei Phe unsubstituiertes oder durch Fluor substituiertes 1,4-Phenylen, Cyc trans-1,4-Cyclohexylen oder 1,4-Cyclohexenylen, Pyr Pyrimidin-2,5-diyl oder Pyridin-2,5-diyl, Dio 1,3-Dioxan-2,5-diyl und G 2-(trans-1,4-Cyclohexyl)-ethyl, Pyrimidin-2,5-diyl, Pyridin-2,5-diyl oder 1,3-Dioxan-2,5-diyl bedeuten.

Vorzugsweise ist einer der Reste L und E Cyc, Phe oder Pyr. E ist-vorzugsweise Cyc, Phe oder Phe-Cyc. Vorzugsweise enthalten die erfindungsgemäßen Medien eine oder mehrere Komponenten ausgewählt aus den Verbindungen der Formeln 1, 2, 3, 4 und 5, worin L und E ausgewählt sind aus der Gruppe Cyc, Phe und Pyr und gleichzeitig eine oder mehrere Komponenten ausgewählt aus den Verbindungen der Formeln 1, 2, 3, 4 und 5, worin einer der Reste L und E ausgewählt ist aus der Gruppe Cyc, Phe und Pyr und der andere Rest ausgewählt ist aus der Gruppe -Phe-Phe-, -Phe-Cyc-, -Cyc-Cyc-, -G-Phe- und -G-Cyc-, und gegebenenfalls eine oder mehrere Komponenten ausgewählt aus den Verbindungen der Formeln 1, 2, 3, 4 und 5, worin die Reste L und E ausgewählt sind aus der Gruppe -Phe-Cyc-, -Cyc-Cyc-, -G-Phe- und -G-Cyc-.

R' und R'' bedeuten in den Verbindungen der Teilformeln 1a, 2a, 3a, 4a und 5a jeweils unabhängig voneinander Alkyl, Alkenyl, Alkoxy, Alkenyloxy oder Alkanoyloxy mit bis zu 8 Kohlenstoffatomen. Bei den meisten dieser Verbindungen sind R' und R'' voneinander verschieden, wobei einer dieser Reste meist Alkyl oder Alkenyl ist. In den Verbindungen der Teilformeln 1b, 2b, 3b, 4b und 5b bedeutet R'' -CN, -$CF_3$, F, Cl oder -NCS; R hat dabei die bei den Verbindungen der Teilformeln 1a bis 5a angegebene Bedeutung und ist vorzugsweise Alkyl oder Alkenyl. Aber auch andere Varianten der vorgesehenen Substituenten in den Verbindungen der Formeln 1, 2, 3, 4 und 5 sind gebräuchlich. Viele solcher Substanzen oder auch Gemische davon sind im Handel erhältlich. Alle diese Substanzen sind nach literaturbekannten Methoden oder in Analogie dazu erhältlich.

Die erfindungsgemäßen Medien enthalten vorzugsweise neben Komponenten aus der Gruppe der Verbindungen 1a, 2a, 3a, 4a und 5a (Gruppe 1) auch Komponenten aus der Gruppe der Verbindungen 1b, 2b, 3b, 4b und 5b (Gruppe 2), deren Anteile vorzugsweise wie folgt sind:
Gruppe 1: 20 bis 90 %, insbesondere 30 bis 90 %,
Gruppe 2: 10 bis 80 %, insbesondere 10 bis 50 %,
wobei die Summe der Anteile der erfindungsgemäßen Verbindungen und der Verbindungen aus den Gruppen 1 und 2 bis zu 100 % ergeben.

Die erfindungsgemäßen Medien enthalten vorzugsweise 1 bis 40 %, insbesondere vorzugsweise 5 bis 30 % an erfindungsgemäßen Verbindungen. Weiterhin bevorzugt sind Medien, enthaltend mehr als 40 %, insbesondere 45 bis 90 % an erfindungegemäßen Verbindungen. Die Medien enthalten vorzugsweise drei, vier oder fünf erfindungsgemäße Verbindungen.

Die Herstellung der erfindungsgemäßen Medien erfolgt in an sich üblicher Weise. In der Regel werden die Komponenten ineinander gelöst, zweckmäßig bei erhöhter Temperatur. Durch geeignete Zusätze können die flüssigkristallinen Phasen nach der Erfindung so modifiziert werden, daß sie in allen bisher bekannt gewordenen Arten von Flüssigkristallanzeigeelementen verwendet werden können. Derartige Zusätze sind dem Fachmann bekannt und in der Literatur ausführlich beschrieben (H. Kelker/R. Hatz, Handbook

of Liquid Crystals, Verlag Chemie, Weinheim, 1980). Beispielsweise können pleochroitische Farbstoffe zur Herstellung farbiger Guest-Host-Systeme oder Substanzen zur Veränderung der dielektrischen Anisotropie, der Viskosität und/oder der Orientierung der nematischen Phasen zugesetzt werden.

Insbesondere eignen sich die erfindungsgemäßen Medien zur Verwendung in MFK-Anzeigen.

Die folgenden Beispiele sollen die Erfindung erläutern, ohne sie zu begrenzen. mp. = Schmelzpunkt, cp. = Klärpunkt. Vor- und nachstehend bedeuten Prozentangaben Gewichtsprozent; alle Temperaturen sind in Grad Celsius angegeben. "Übliche Aufarbeitung" bedeutet: man gibt Wasser hinzu, extrahiert mit Methylenchlorid, trennt ab, trocknet die organische Phase, dampft ein und reinigt das Produkt durch Kristallisation und/oder Chromatographie.

Es bedeuten ferner:

K: Kristallin-fester Zustand, S: smektische Phase (der Index kennzeichnet den Phasentyp), N: nematischer Zustand, Ch: cholesterische Phase, I: isotrope Phase. Die zwischen zwei Symbolen stehende Zahl gibt die Umwandlungstemperatur in Grad Celsius an.

| | |
|---|---|
| DAST | Diethylaminoschwefeltrifluorid |
| DCC | Dicyclohexylcarbodiimid |
| DDQ | Dichlordicyanobenzochinon |
| DIBALH | Diisobutylaluminiumhydrid |
| DMSO | Dimethylsulfoxid |
| KOT | Kalium-tertiär-butanolat |
| THF | Tetrahydrofuran |
| pTSOH | p-Toluolsulfonsäure |

Beispiel 1

trans-4-Trifluormethylcyclohexancarbonsäure

Ein Gemisch aus 0,47 mol 4-Trifluormethylbenzoesäureethylester (hergestellt aus 4-Trifluormethylbenzoylchlorid, Ethanol und Pyridin), 1000 ml Ethanol und 20 g Rhodium/Aktivkohle (5 %) wird unter einem Druck von 5 bar, bei einer Temperatur von 60 °C 5 Stunden hydriert. Der nach Abfiltrieren des Katalysators und Entfernen des Lösungsmittels erhaltene 4-Trifluormethylcyclohexancarbonsäureethylesters (cis/trans = 87 %/13 %) wird ohne vorherige Reinigung in 300 ml Wasser suspendiert mit 70 g 30%iger Natronlauge versetzt, kurz zum Sieden erhitzt und 18 h bei Raumtemperatur gerührt. Nach Ansäuern mit Salzsäure erhält man die Carbonsäure als cis/trans-Gemisch.

Ein Gemisch aus 0,38 mol dieser Säure und 150 ml Thionylchlorid wird 48 h zum Sieden erhitzt. Nach Abdestillation des überschüssigen Thionylchlorids werden 500 ml Wasser und 100 ml einer 30%igen Natronlauge hinzugefügt und das Reaktionsgemisch 2 h bei 60 °C gerührt. Nach Ansäuern und Umkristallisation aus Petrolether erhält man die reine trans-Carbonsäure mit einem Schmelzpunkt von 155 °C.

Beispiel 2

trans-4-Trifluormethylcyclohexanol

Ein Gemisch aus 0,3 mol 4-Trifluormethylphenol, 1000 ml Ethanol und 20 g Rhodium/Aktivkohle (5 %) wird bei einem Druck von 5 bar und einer Temperatur von 60 °C 2 Stunden hydriert. Nach Abfiltrieren der festen Bestandteile und Abdestillation des Lösungsmittels erhält man das Cyclohexanol als cis/trans-Gemisch (30 %/70 %). Aus diesem wird die reine trans-Verbindung durch Erhitzen mit Aluminiumtriisopropylat in Toluol in Gegenwart von 3,3-Dimethyl-2-butanon und anschließendes Umkristallisieren, erhalten.

Beispiel 3

trans-4-Trifluormethyl-1-formylcyclohexan

Zu 100 ml einer Lösung von Isobutylmagnesiumbromid in Ether (1 mol/l) und 0,3 mmol Dichlorbis[$\pi$-cyclopentadienyl]-titan wird bei 0 °C 0,05 mol von trans-4-Trifluormethylcyclohexancarbonsäure (hergestellt nach Beispiel 1) getropft und 4 h bei Raumtemperatur gerührt. Nach Zugabe von 30 ml einer 4 normalen Salzsäure und üblicher Aufarbeitung erhält man das Produkt als farbloses Öl.

Beispiel 4

trans-4-Trifluormethylcyclohexancarbonsäure-(trans,trans-4'-propylbicyclohexyl)-ester.

Ein Gemisch aus 0,005 mol trans-4-Trifluormethylcyclohexancarbonsäure, 0,005 mol trans,trans-4-Propylbicyclohexyl-4'-ol, 100 ml Dichlormethan, 0,005 mol 4-N,N-Dimethylaminopyridin und 0,007 mol DCC wird 2 h bei Raumtemperatur gerührt. Nach Abfiltration der festen Bestandteile und üblicher Aufarbeitung erhält man das Produkt als farblosen Feststoff, K 89 $S_B$ 165 I, Δn • 0,065, Δε + 10,1.

Analog werden hergestellt

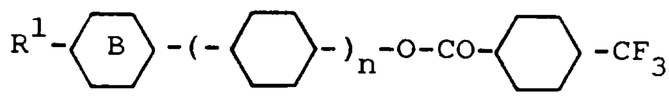

| $R^1$ | n | B 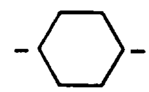 | |
|---|---|---|---|
| $C_5H_{11}$ | 1 | 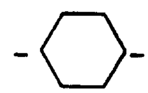 | |
| $C_7H_{15}$ | 1 | 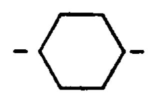 | |
| $C_3H_7$ K 45 I Δε + 9,4 Δn 0,012 | 0 | 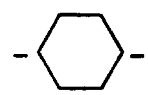 | |
| $C_5H_{11}$ | 0 | 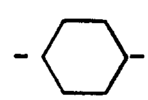 | |
| $C_7H_{15}$ | 0 | 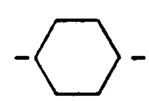 | |
| $C_5H_{11}$ | 0 | 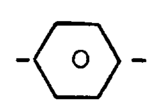 | K 48 I |

Beispiel 5

trans-4-Pentylcyclohexancarbonsäure-(trans-4-trifluormethylcyclohexyl)ester

0,005 mol trans-4-Pentylcyclohexancarbonsäure werden mit 0,005 mol trans-4-Trifluormethylcyclohexanol (hergestellt nach Beispiel 2) analog Beispiel 4 verestert. Man erhält das Produkt als farblosen Feststoff.

Analog werden hergestellt:

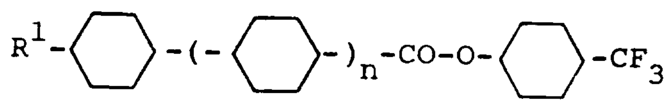

| $R^1$ | n |
|---|---|
| $C_3H_7$ | 0 |
| $C_7H_{15}$ | 0 |
| $C_3H_7$ | 1 |
| $C_5H_{11}$ | 1 |
| $C_7H_{15}$ | 1 |

Beispiel 6

trans-4-Heptylcyclohexylmethyl-(trans-4-trifluormethylcyclohexyl)-ether

Zu einem Gemisch aus 0,1 mol Natrium-(trans-4-trifluormethylcyclohexanolat) (hergestellt aus 0,1 mol trans-4-Trifluormethylcyclohexanol und 0,1 mol Natriumhydrid) und 200 ml THF gibt man bei 0 °C ein Gemisch aus 0,11 mol trans-4-Heptylcyclohexylmethylbromid und 80 ml THF. Nach 3stündigem Rühren bei 60 °C und nach üblicher Aufarbeitung erhält man das Produkt als farblosen Feststoff.

Analog werden hergestellt:

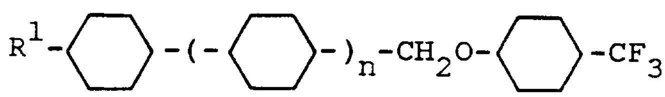

| $R^1$ | n |
|---|---|
| $C_3H_7$ | 0 |
| $C_5H_{11}$ | 0 |
| $C_3H_7$ | 1 |
| $C_5H_{11}$ | 1 |
| $C_7H_{15}$ | 1 |

Mit trans-4-Trifluormethylcyclohexylmethylbromid (hergestellt aus trans-4-Trifluorcyclohexancarbonsäure durch Reduktion mit Lithiumaluminiumhydrid und anschließende Bromierung mit Phosphortribromid) und trans-4-Alkyl-Cyclohexanolen erhält man analog:

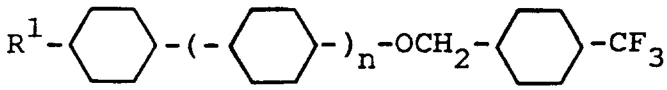

| $R^1$ | n |
|---|---|
| $C_3H_7$ | 0 |
| $C_5H_{11}$ | 0 |
| $C_7H_{15}$ | 0 |
| $C_3H_7$ | 1 |
| $C_5H_{11}$ | 1 |
| $C_7H_{15}$ | 1 |

Beispiel 7

1-(trans-4-Propylcyclohexyl)-2-(trans-4-trifluormethylcyclohexyl)-ethen

Zu einem Gemisch aus 0,2 mol Natriumhydrid und 100 ml DMSO gibt bei 0 °C ein Gemisch aus 0,2 mol (trans-4-Propylcyclohexylmethyl)-triphenylphosphoniumbromid (hergestellt aus trans-4-Propylcyclohexylmethylbromid und Triphenylphosphin) und 150 ml DMSO. Nach 10minütigem Rühren bei Raumtemperatur wird ein Gemisch aus 0,2 mol trans-4-Trifluormethyl-1-formylcyclohexan (hergestellt nach Beispiel 3) und 50 ml DMSO hinzugetropft. Nach 1stündigem Erwärmen auf 65 °C wird wie üblich aufgearbeitet. Man erhält das Produkt als farblosen Farbstoff.

Analog werden hergestellt:

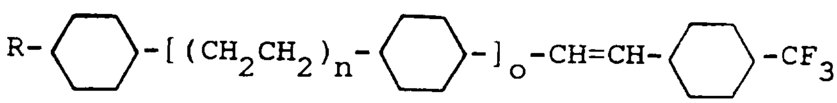

| R | O | n |
|---|---|---|
| $C_2H_5$ | 0 | 0 |
| $C_5H_{11}$ | 0 | 0 |
| $C_7H_{15}$ | 0 | 0 |
| $C_3H_7$ | 1 | 0 |
| $C_5H_{11}$ | 1 | 0 |
| $C_7H_{15}$ | 1 | 0 |
| $C_3H_7$ | 1 | 1 |
| $C_5H_{11}$ | 1 | 1 |
| $C_7H_{15}$ | 1 | 1 |

Beispiel 8

1-(trans-4-Propylcyclohexyl)-2-(trans-4-trifluormethylcyclohexyl)-ethan

Ein Gemisch aus 0,1 mol 1-(trans-4-Propylcyclohexyl)-2-(trans-4-trifluormethylcyclohexyl)-ethen, 200 ml Methanol und 5 g Palladium/Aktivkohle (15 %) wird unter einem Druck von 2 bar bei einer Temperatur von 50 °C 2 Stunden hydriert. Nach üblicher Aufarbeitung erhält man das Produkt als farblosen Feststoff.

Analog werden hergestellt:

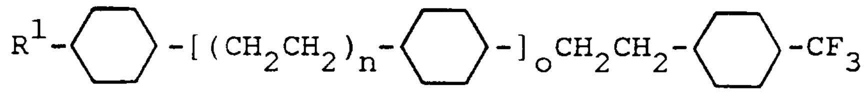

| $R^1$ | O | n |
|---|---|---|
| $C_5H_{11}$ | 0 | 0 K 48 I, Δε +6,3, Δn 0,025 |
| $C_7H_{15}$ | 0 | 0 |
| $C_3H_7$ | 1 | 0 K 157 $S_B$ (156) N 158,5 I, Δε +8,7, Δn 0,054 |
| $C_5H_{11}$ | 1 | 0 |
| $C_7H_{15}$ | 1 | 0 |
| $C_3H_7$ | 1 | 1 |
| $C_5H_{11}$ | 1 | 1 |
| $C_7H_{15}$ | 1 | 1 |

Beispiel 9

trans,trans-4'-Propyl-4-trifluormethylbicyclohexan

Ein Gemisch aus 0,35 mol trans,trans-4'-Pentylbicyclohexyl-4-carbonsäure und 0,9 mol Schwefeltetrafluorid werden in Autoklaven 10 Tage auf 70 °C erwärmt. Das Produkt wird in Pentan aufgenommen, mit Wasser gewaschen, getrocknet und eingedampft. Nach Destillation und Kristallisation erhält man das Produkt K 43 I.

Analog werden hergestellt

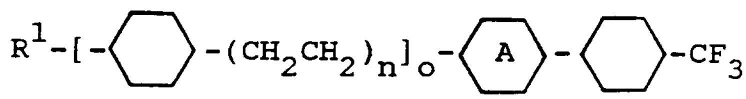

| $R^1$ | n | o | 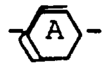 |
|---|---|---|---|
| $C_5H_{11}$ | 0 | 0 | 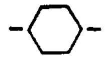 |
| $C_7H_{15}$ | 0 | 0 | 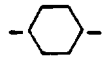 |
| $C_3H_7$ | 0 | 1 | 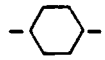 |
| $C_5H_{11}$ | 0 | 1 | 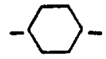 |
| $C_7H_{15}$ | 0 | 1 | 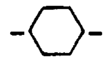 |
| $C_3H_7$ | 1 | 1 | 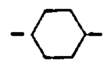 |
| $H_2C{=}CH{-}(CH_2)_3{-}$ | 0 | 0 | 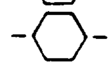 |
| $H_2C{=}CH{-}(CH_2)_2{-}$ | 0 | 0 | 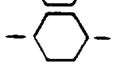 |
| $H_3C{-}CH{=}CH{-}CH_2{-}$ | 0 | 0 | 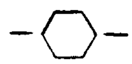 |
| $C_5H_{11}$ | 1 | 1 | 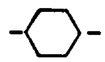 |
| $C_7H_{15}$ | 1 | 1 | 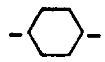 |
| $C_3H_7$ | 0. | 0 | 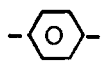 |
| $C_5H_{11}$ | 0 | 0 | 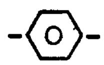 |
| $C_7H_{15}$ | 0 | 0 | 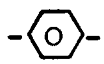 |
| $C_2H_5O$ | 0 | 0 | 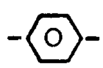 |

Beispiel 10

4-(trans-4-Propylcyclohexyl)-1-trifluormethylcyclohex-1-en

Ein Gemisch aus 10 mmol 4-(trans-4-Propylcyclohexyl)-cyclohexanon, 11 mmol Trimethyl-(trifluormethyl)-silan, 10 mmol Tetrabutylammoniumfluorid und 50 ml THF wird 2 Stunden bei 0 °C gerührt. Nach üblicher Aufarbeitung wird das Rohprodukt gelöst, in 20 ml THF mit 10 mmol DAST versetzt und zum Sieden erhitzt. Nach üblicher Aufarbeitung wird das Rohprodukt durch Chromatographie und Kristallisation gereinigt.

Analog werden hergestellt:

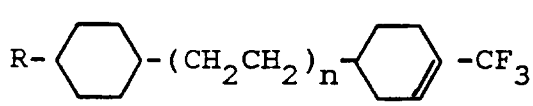

| R | n |
|---|---|
| $C_5H_{11}$ | 0 |
| $C_7H_{15}$ | 0 |
| $C_3H_7$ | 1 |
| $C_5H_{11}$ | 1 |
| $C_7H_{15}$ | 1 |

Beispiel 11

2-(trans-4-Trifluormethylcyclohexyl)-5-propyl-1,3-dioxan

Ein Gemisch aus 0,02 mol trans-4-Trifluormethyl-1-formylcyclohexan (hergestellt nach Beispiel 3), 0,02 mol 2-Propylpropandiol, 30 ml Toluol und 0,5 g pTsOH wird 4 Stunden am Wasserabscheider erhitzt. Man erhält das reine Produkt nach üblicher Aufarbeitung und Kristallisation.

Analog werden hergestellt:

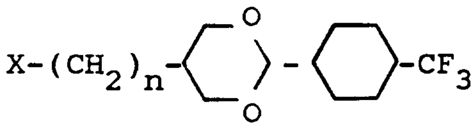

| X | n |
|---|---|
| H | 5 , K 41 $S_\beta$ 42 I |
| H | 7 |
| $H_2C=CH$ | 1 |
| $CH_3$-CH=CH | 1 |
| $H_2C=CH$ | 2 |
| $H_2C=CH$ | 3 |
| $H_2C=CH$ | 4 |
| $H_2C=CH$ | 5 |
| $H_2C=CH$ | 6 |
| $H_3CO$ | 1 |
| $H_3CO$ | 2 |
| $H_3CO$ | 3 |
| $H_3CO$ | 4 |
| $H_3CO$ | 5 |
| $H_3CO$ | 5 |

| X | n |
|---|---|
| F | 1 |
| F | 2 |
| F | 3 |
| F | 4 |
| F | 5 |
| F | 6 |

Beispiel 12

2-(trans-4-Trifluormethylcyclohexyl)-5-(trans-4-pentylcyclohexyl)-1,3-dioxan

Ein Gemisch aus 0,02 mol trans-4-Trifluormethyl-1-formylcyclohexan (hergestellt nach Beispiel 3), 0,02 mol 2-(trans-4-Pentylcyclohexyl)-propandiol (hergestellt nach DE-32 27 916), 30 ml Toluol und 0,5 g pTSOH wird 6 Stunden am Wasserabscheider erhitzt. Man erhält das Produkt nach üblicher Aufarbeitung und Kristallisation.

Analog werden hergestellt:

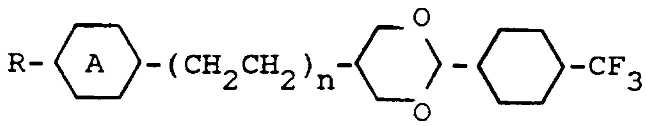

| R | n | 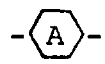 |
|---|---|---|
| $C_3H_7$ | 0 | 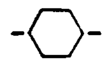 |
| $C_7H_{15}$ | 0 | 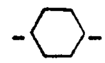 |
| $C_3H_7$ | 0 | 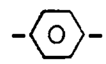 |
| $C_5H_{11}$ | 0 | 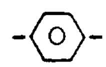 |
| $C_7H_{15}$ | 0 | 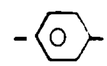 |

| R | n | 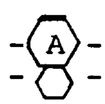 |
|---|---|---|
| $C_3H_7$ | 1 | |
| $C_5H_{11}$ | 1 | 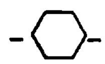 |
| $C_7H_{15}$ | 1 | 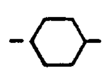 |
| $C_5H_{11}$ | 1 | 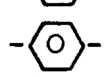 |

Beispiel 13

1-(4'-Pentyl-2'-fluorbiphenyl-4-yl)-2-(trans-4-trifluormethylcyclohexyl)-ethan

Aus 0,2 mol 4'-Pentyl-2'-fluorbiphenyl-4-yl-methylbromid und 0,2 mol trans-4-Trifluormethylformylcyclohexan (hergestellt nach Beispiel 3) erhält man analog Beispiel 7 1-(4'-Pentyl-2-fluorbiphenyl-4-yl)-2-(trans-4-trifluormethylcyclohexyl)-ethen. Dieses wird analog Beispiel 8 in Methanol mit Palladium/Aktivkohle (15 %) hydriert. Mach üblicher Aufarbeitung und Kristallisation erhält man das reine Produkt.

Analog werden hergestellt:

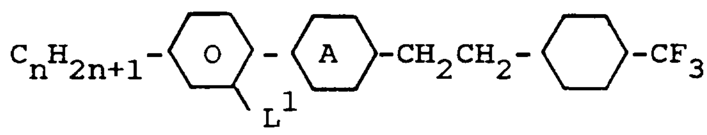

| n | $L^1$ | 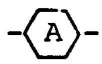 |
|---|---|---|
| 3 | F | 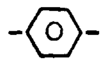 |
| 7 | F | 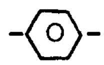 |
| 3 | H | 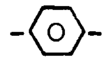 |
| 5 | H | 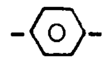 |
| 7 | H | 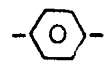 |
| 3 | F | 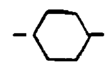 |

| n | $L^1$ | 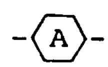 |
|---|---|---|
| 5 | F | 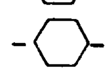 |
| 7 | F | 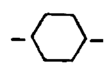 |
| 3 | H | 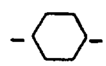 |
| 5 | H | 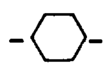 |
| 7 | H | 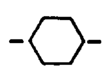 |

Beispiel 14

trans-4-(2-Ethylpyrimidin-5-yl)-trifluormethylcyclohexan

Ein Gemisch aus 0,1 mol 2-(Diethoxymethyl)-butyraldehyd (erhältlich aus 2-Ethylmalonsäurediethylester), 0,1 mol trans-4-Trifluormethylcyclohexylcarbamidin-Hydrochlorid (erhältlich aus trans-4-Trifluormethyl-1-formylcyclohexan, vgl. Beispiel 3, durch Behandeln mit Hydroxylamin-O-sulfonsäure in wässriger Lösung bei 65 °C und anschließende Umsetzung mit alkoholischer Salzsäure) und 100 ml Dimethylformamid wird 4 h auf 130 °C erhitzt und nach Zugabe von weiteren 100 ml Dimethylformamid nochmals 16 h auf 130 °C erhitzt. Nach üblicher Aufarbeitung erhält man das reine Produkt durch Umkristallisation.

Analog werden hergestellt:

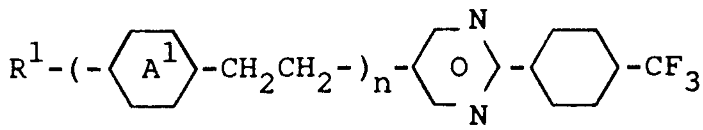

| $R^1$ | n | 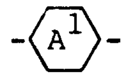 |
|---|---|---|
| $C_3H_7$ | 0 | - |
| $C_5H_{11}$ | 0 | - |
| $C_7H_{15}$ | 0 | - |
| $C_3H_7$ | 1 | 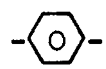 |
| $C_5H_{11}$ | 1 | 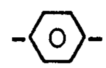 |
| $C_7H_{15}$ | 1 | 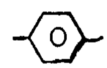 |
| $C_3H_7$ | 1 | 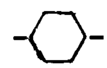 |

Mischungsbeispiel A

Es wird ein flüssigkristallines Medium, welches aus 90 Gew.% einer Basismischung (A) bestehend aus
24 Gew.% p-(trans-Propylcyclohexyl)-benzonitril
36 Gew.% p-(trans-Pentylcyclohexyl)-benzonitril
25 Gew.% p-(trans-Heptylcylcohexyl)-benzonitril
15 Gew.% 4'-(trans-4-Pentylcyclohexyl)-4-cyanbiphenyl und
10 % trans-4-Trifluormethylcyclohexancarbonsäure-(trans,trans-4-propylbicyclohexyl)-ester
besteht, hergestellt.

Mischungsbeispiel B

Es wird ein flüssigkristallines Medium, welches aus 90 Gew.% der Basismischung (A) und
10 Gew.% 2-(trans,trans-4'-Propylbicyclohexyl-4-yl)-1-(trans-4-trifluormethylcyclohexyl)-ethan
besteht, hergestellt.

Mischungsbeispiel C

Es wird ein flüssigkristallines Medium, welches aus 90 Gew.% der Basismischung (A) und
10 Gew.% 2-(trans-4-Pentylcyclohexyl)-1-(trans-4-trifluormethylcyclohexyl)-ethan
besteht, hergestellt.

## Patentansprüche

1. Trifluormethylcyclohexan-Derivate der Formel I

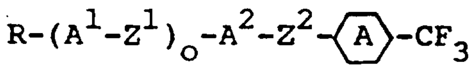

I

wobei

R ein unsubstituierter oder mit CN oder mit mindestens einem Halogen substituierter Alkyl- oder Alkenylrest mit bis zu 18 C-Atomen, worin eine oder mehrere nicht benachbarte $CH_2$-Gruppen durch einen Rest ausgewählt aus der Gruppe -O-, -S-, -CO-, -O-CO-, -CO-O- und -C≡C- ersetzt sein können,

$A^1$ und $A^2$ jeweils unabhängig voneinander

a) einen 1,4-Phenylenrest, worin eine oder zwei CH-Gruppen durch N ersetzt sein können,

b) einen 1,4-Cyclohexylenrest, worin eine oder zwei nicht benachbarte $CH_2$-Gruppen durch -O- oder -S- ersetzt sein können,

c) einen 1,4-Cyclohexenylen-, einen Piperidin-1,4-diyl-, einen 1,4-Bicyclo[2,2,2]-octylen- oder einen Naphthalin-2,6-diylrest

wobei die Reste a) und b) ein oder mehrfach durch Halogenatome, Cyano- und/oder Methylgruppen substituiert sein können,

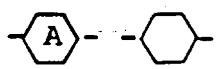 oder 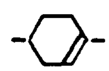

$Z^1$ und $Z^2$ jeweils unabhangig voneinander -CO-O-, -O-CO-, $-CH_2CH_2-$, $-CH(CN)-CH_2-$, $-CH_2-CH(CN)-$, -CH = CH-, $-OCH_2-$, $-CH_2O-$, -CH = N-, -N = CH-, -NO = N-, -N = NO-, -N = N- oder eine Einfachbindung, und

o 0, 1 oder 2 bedeuten.

2. Trifluormethylcyclohexan-Derivate nach Anspruch 1, dadurch gekennzeichnet, daß

$A^1$ und $A^2$ jeweils unabhängig voneinander einen 1,4-Cyclohexylenrest, worin eine oder zwei nicht benachbarte $CH_2$-Gruppen durch -O- oder -S- ersetzt sein können.

3. Trifluormethylcyclohexan-Derivate nach Anspruch 1 oder 2, dadurch gekennzeichnet, daß

$Z^2$ -O-CO- oder $-CH_2CH_2-$

bedeutet.

4. Trifluormethylcyclohexan-Derivate nach Anspruch 1 oder 2 der Formel I1,

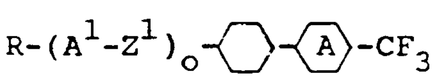

I1

worin R, $A^1$, - A -, $Z^1$ und o die angegebene Bedeutung besitzen.

5. 2-(Trifluormethycyclohexyl)-Dioxan-Derivate nach Anspruch 1 oder 2 der Formel I2,

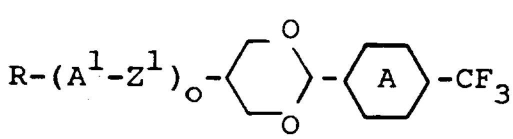

worin R, $A^1$ - A -, $Z^1$ und O die angegebene Bedeutung besitzen.

**6.** 2-(Trifluormethylcyclohexyl)-Dioxan-Derivate nach Anspruch 5 der Formel I2a

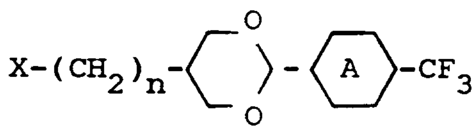

worin

| | |
|---|---|
| X | F, Cl, $CH=CH_2$, $CH=CH-C_mH_{2m+1}$ $O-C_mH_{2m+1}$,C≡CH oder H, und |
| m und n | jeweils unabhängig voneinander 0 bis 10 |

bedeuten.

**7.** Trifluormethylcyclohexan-Derivate nach einem der Ansprüche 1 bis 6, dadurch gekennzeichnet, daß die 1,4-Cyclohexylenreste in der trans-Konfiguration vorliegen.

**8.** Verwendung der Verbindungen der Formel I als Komponenten flüssigkristalliner Medien für elektrooptische Anzeigeelemente.

**9.** Flüssigkristallines Medium mit mindestens zwei Komponenten, dadurch gekennzeichnet, daß eine oder mehrere Komponenten eine Verbindung der Formel I sind.

**10.** Elektrooptische Anzeige, dadurch gekennzeichnet, daß sie als Dieelektrikum ein flüssigkristallines Medium nach Anspruch 9 enthält.

**11.** Matrix-Flüssigkristallanzeige nach Anspruch 8, dadurch gekennzeichnet, daß sie als Dielektrikum ein flüssigkristallines Medium nach Anspruch 9 enthält.

**12.** 4-Substituierte 1-Trifluormethylcyclohexane der Formel II,

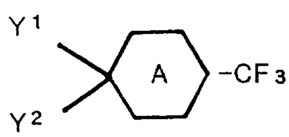

II

worin

| | |
|---|---|
| $Y^1$ | -(CH2)n-$Y^3$, -CHO oder CN, |
| $Y^2$ | H, |
| $Y^3$ | OH oder Halogen, und |
| n | 1 oder 2 bedeuten. |

## Claims

**1.** Trifluoromethylcyclohexane derivatives of the formula I

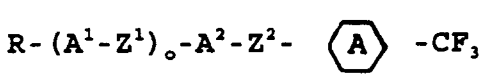

I

where

| | |
|---|---|
| R | is an alkyl or alkenyl radical having up to 18 C atoms which is unsubstituted or substituted by CN or by at least one halogen, and in which one or more non-adjacent $CH_2$ groups may be replaced by a radical selected from the group comprising -O-, -S-, -CO-, -O-CO-, -CO-O- and -C≡C-, |

$A^1$ and $A^2$ are each, independently of one another,

a) a 1,4-phenylene radical, in which one or two CH groups may be replaced by N,

b) a 1,4-cyclohexylene radical, in which one or two non-adjacent $CH_2$ groups may be replaced by -O- or -S-,

c) a 1,4-cyclohexenylene, piperidine-1,4-diyl, 1,4-bicyclo[2,2,2]-octylene or naphthalene-2,6-diyl radical,

it being possible for the radicals a) and b) to be monosubstituted or polysubstituted by halogen atoms or cyano and/or methyl groups,

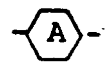

is

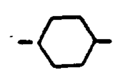 or 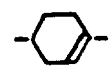

$Z^1$ and $Z^2$ are each, independently of one another, -CO-O-, -O-CO-, $-CH_2CH_2-$, $-CH(CN)-CH_2-$, $-CH_2-CH(CN)-$, -CH = CH-, $-OCH_2-$, $-CH_2O-$, -CH = N-, -N = CH-, -NO = N-, -N = NO-, -N = N- or a single bond, and

o is 0, 1 or 2.

**2.** Trifluoromethylcyclohexane derivatives according to Claim 1, characterized in that

$A^1$ and $A^2$ are each, independently of one another, a 1,4-cyclohexylene radical, in which one or two non-adjacent $CH_2$ groups may be replaced by -O- or -S-.

**3.** Trifluoromethylcyclohexane derivatives according to Claim 1 or 2, characterized in that

$Z^2$ is -O-CO- or $-CH_2CH_2-$.

**4.** Trifluoromethylcyclohexane derivatives according to Claim 1 or 2, of the formula I1

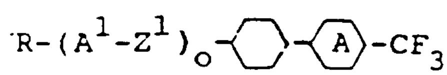 I1

in which R, $A^1$, - A - , $Z^1$ and o are as defined above.

**5.** 2-(Trifluoromethycyclohexyl)dioxane derivatives according to Claim 1 or 2, of the formula I2

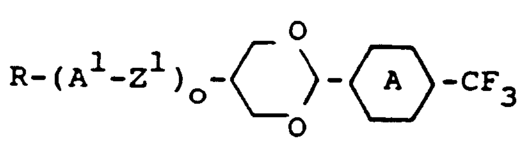

in which R, $A^1$, - A -, $Z^1$ and O are as defined above.

**6.** 2-(Trifluoromethylcyclohexyl)dioxane derivatives according to Claim 5, of the formula I2a

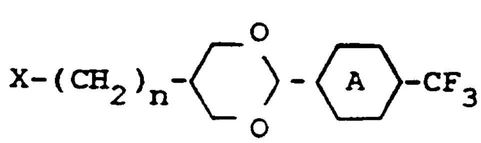

in which

X is F, Cl, $CH=CH_2$, $CH=CH-C_mH_{2m+1}$, $O-C_mH_{2m+1}$, $C\equiv CH$ or H, and

m and n are each, independently of one another, 0 to 10.

**7.** Trifluoromethylcyclohexane derivatives according to one of Claims 1 to 6, characterized in that the 1,4-cyclohexylene radicals are in the trans-configuration.

**8.** Use of the compounds of the formula I as components of liquid-crystalline media for electrooptical display elements.

**9.** Liquid-crystalline medium having at least two components, characterized in that one or more components is a compound of the formula I.

**10.** Electrooptical display, characterized in that it contains, as a dielectric, a liquid-crystalline medium according to Claim 9.

**11.** Matrix liquid-crystal display according to Claim 8, characterized in that it contains, as a dielectric, a liquid-crystalline medium according to Claim 9.

**12.** 4-substituted 1-trifluoromethylcyclohexanes of the formula II,

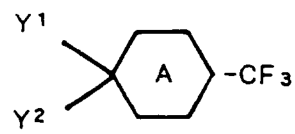

II

in which

$Y^1$ is $-(CH2)n-Y^3$ , -CHO or CN,

$Y^2$ is H,

$Y^3$ is OH or halogen, and

n is 1 or 2.

## Revendications

**1.** Dérivés du trifluorométhylcyclohexane répondant à la formule I

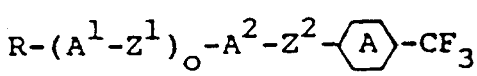

I

dans laquelle

R représente un groupe alkkyle ou alcényle contenant jusqu'à 18 atomes de C, non substitué ou substitué par CN ou par au moins un halogène et dans lequel un ou plusieurs groupes $CH_2$ non voisins peuvent être remplacés par un pont choisi parmi -O-, -S-, -CO-, -O-CO-, -CO-O- et -C=C-,

$A^1$ et $A^2$ représentent chacun, indépendamment l'un de l'autre,

a) un groupe 1,4-phénylène dans lequel un ou deux groupes CH peuvent être remplacés par N,

b) un groupe 1,4-cyclohexylène dans lequel un ou deux groupes $CH_2$ non voisins peuvent être remplacés par -O- ou -S-,

c) un groupe 1,4-cyclohexénylène, un groupe pipéridine- 1,4-diyle, un groupe 1,4-bicyclo[2,2,2]-octylène ou un groupe naphtalène-2,6-diyle,

les groupes a) et b) pouvant eux-mêmes porter un ou plusieurs substituants choisis parmi les atomes d'halogènes, les groupes cyano et/ou méthyle,

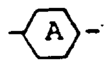

représente

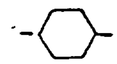 ou 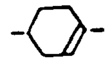

$Z^1$ et $Z^2$ représentent chacun, indépendamment de l'autre, -CO-O-, -O-CO-, $-CH_2CH_2-$, $-CH(CN)-CH_2-$, $-CH_2-CH(CN)-$, -CH = CH-, $-OCH_2-$, $-CH_2O-$, -CH = N-, -N = CH-, -NO = N-, -N = NO-, -N = N- ou une liaison simple et
o est égal à 0, 1 ou 2.

**2.** Dérivés du trifluorométhylcyclohexane selon revendication 1, caractérisés en ce que $A^1$ et$A^2$ représentent chacun, indépendamment l'un de l'autre, un groupe 1,4-cyclohexylène dans lequel un ou deux groupes $CH_2$ non voisins peuvent être remplacés par -O- ou -S-.

**3.** Dérivés du trifluorométhylcyclohexane selon revendication 1 ou 2, caractérisés en ce que $Z^2$ représente -O-CO- ou $-CH_2CH_2-$.

**4.** Dérivés du trifluorométhylcyclohexane selon revendication 1 ou 2, de formule I1

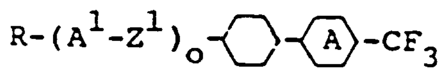 I1

dans laquelle R, $A^1$, -A-, $Z^1$ et o ont les significations indiquées ci-dessus.

**5.** Dérivés du 2-(trifluorométhylcyclohexyl)-dioxanne selon revendication 1 ou 2, de formule I2

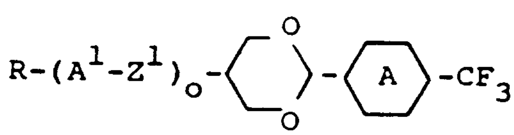

dans laquelle R, $A^1$, -A-, $Z^1$ et o ont les significations indiquées ci-dessus.

**6.** Dérivés du 2-(trifluorométhylcyclohexyl)-dioxanne selon revendication 5, de formule I2a

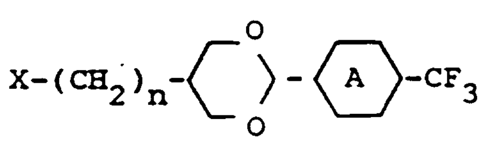

dans laquelle
X représente
F, Cl, $CH = CH_2$, $CH = CH-C_mH_{2m+1}$ $O-C_mH_{2m+1}$, C≡CH ou H,
et m et n sont égaux chacun, indépendamment l'un de l'autre, à un nombre allant de 0 à 10.

**7.** Dérivés du trifluorométhylcyclohexane selon une des revendications 1 à 6, caractérisés en ce que les groupes 1,4-cyclohexylène sont en configuration trans.

**8.** Utilisation des composés de formule I en tant que composants de milieux à cristaux liquides pour des éléments d'affichage électro-optique.

**9.** Milieu à cristaux liquides à au moins deux composants, caractérisé en ce qu'un ou plusieurs composants consistent en composés de formule I.

**10.** Affichage électro-optique caractérisé en ce qu'il contient en tant que diélectrique un milieu à cristaux liquides selon revendication 9.

**11.** Affichage à cristaux liquides à matrice selon revendication 8, caractérisé en ce qu'il contient en tant que diélectrique un milieu à cristaux liquides selon revendication 9.

**12.** 1-trifluorométhylcyclohexanes substitués en position 4, répondant à la formule II

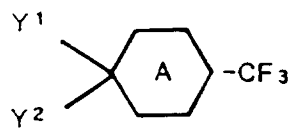

II

dans laquelle
$Y^1$ représente -(CH2)n-$Y^3$, -CHO ou CN,
$Y^2$ représente H,
$Y^3$ représente OH ou un halogène et
n est égal à 1 ou 2.